# EUROPÄISCHE PATENTSCHRIFT

(11) **EP 3 049 036 B1**
(45) Veröffentlichungstag und Bekanntmachung des Hinweises auf die Patenterteilung: **25.04.2018**
(21) Anmeldenummer: 14824384.3
(22) Anmeldetag: 02.12.2014
(51) Int. Cl.: A61F 5/01, A61F 5/32, A61F 5/34

(54) **BEHANDLUNGSGERÄT ZUR DEHNUNG DES KARPALKANALS DER MENSCHLICHEN HAND**
TREATMENT DEVICE FOR STRETCHING THE CARPAL TUNNEL OF THE HUMAN HAND
DISPOSITIF DE TRAITEMENT POUR ÉTIRER LE CANAL CARPIEN DE LA MAIN HUMAINE

(30) Priorität: 05.12.2013 DE 102013113564
(43) Veröffentlichungstag der Anmeldung: 03.08.2016
(73) Patentinhaber: Heyl, Dieter, 77855 Achern (DE)
(72) Erfinder: Heyl, Dieter, 77855 Achern (DE)
(74) Vertreter: Lemcke, Brommer & Partner Patentanwälte Partnerschaft mbB
(86) Internationale Anmeldenummer: PCT/EP2014/076213
(87) Internationale Veröffentlichungsnummer: WO 2015/082451

(56) Entgegenhaltungen:
- US-A- 6 146 347
- US-A1- 2005 228 323
- US-B1- 6 217 536
- US-B1- 6 315 748
- US-B1- 6 517 501
- US-B1- 6 716 185

## Beschreibung

Die vorliegende Erfindung betrifft ein Behandlungsgerät zur Dehnung des Karpalkanals der menschlichen Hand.

Der Karpalkanal, häufig auch als Karpaltunnel bezeichnet, ist eine von Bindegewebe umschlossene Röhre vom Unterarm zur Hand auf der Handflächenseite des Handgelenks. Der "Boden" und die Seitenwände des Kanals werden von den Handwurzelknochen gebildet. Das "Dach" des Kanals bildet das etwa 4 bis 5 cm breite Karpalband, dass sich quer zwischen den Handwurzelknochen auspannt. Durch den Kanal verlaufen mehrere Beugesehnen sowie der Medianus-Nerv, der unter anderem die Bewegung der Finger und des Daumens steuert und auch für deren Empfindungsfähigkeit zuständig ist. Durch eine Einengung des Karpalkanals beispielsweise aufgrund arthrotischer Veränderungen oder überlastungsbedingte Schwellungszustände, kann der Medianus-Nerv geschädigt werden. Eine solche Schädigung wird als Karpaltunnelsyndrom bezeichnet und kann sich je nach Progredienz durch Missempfindungen, Taubheitsgefühl, Schmerzen und/oder motorischen Einschränkungen äußern.

Eine Behandlung des Karpaltunnelsyndroms erfolgt häufig operativ durch ein Durchtrennen des Karpalbandes, um den Druck auf den Medianus-Nerv zu beseitigen und diesem mehr Raum zu geben. Daneben bestehen konservative Therapiemöglichkeiten, die das Tragen spezieller Schienen oder Stützverbände umfassen. Als weitere Behandlungsalternative wurden spezielle Schienen vorgeschlagen, die den Karpalkanal öffnen, indem dieser gedehnt und dadurch die querliegenden Bänder verlängert werden, so dass hierdurch mehr Raum geschaffen wird.

Aus der Schrift US 6,146,347 ist eine solche Schiene zur Dehnung des Karpalkanals bekannt. Diese wird um die Hand gelegt und soll durch Aufblasen zweier Luftkissen, die sich auf der Handrückseite befinden, den Karpalkanal dehnen. Die Kräfte, die hierbei auf das Karpalband ausgeübt werden, sind bei dieser Schiene jedoch relativ gering, so dass die Einsatzmöglichkeiten des Geräts eingeschränkt sind.

Ein weiteres Gerät zur Dehnung des Karpalkanals ist in der Schrift US 2004/0210169 beschrieben. Es besitzt eine ringförmig um eine zu behandelnde Hand greifende Mechanik, welche über entsprechende Polster Druck auf Handballen und Handrücken ausübt. Das Gerät ist jedoch relativ aufwändig und kompliziert aufgebaut und damit teuer in der Herstellung. Außerdem hat die gezeigte Apparatur ein hohes Gewicht und ist entsprechend unkomfortabel in der Benutzung. Darüber hinaus muss, um höhere Dehnkräfte zu erzielen, die senkrechte Krafteinleitung recht hoch sein, was nach kurzer Zeit zu Schmerzen führen kann.

Aus der Schrift US 6,315,748 ist eine Bandage gezeigt, welche über den Handrücken gelegt wird und über elastische, selbstklebende Bänder verfügt, die auf die Handinnenseite aufgeklebt werden. Die gezeigte Bandage ermöglicht jedoch keine kontinuierliche Krafteinleitung, da die Klebebänder starr aufgeklebt werden. Außerdem fehlt eine geeignete Gegenkraft zur wirksamen Verformung des Karpalbandes.

Außerdem ist aus der Schrift US6217536 B1 ein Behandlungsgerät zur Vorbeugung oder Behandlung eines Karpaltunnelsyndroms bekannt, welches ein Druckkissen zur Auflage auf die Handballen einer zu behandelnden Hand und eine über den Handrücken zu legende Dehnvorrichtung mit Abstandshaltern aufweist, über die Zugbänder mit Klettverschlüssen zu dem Druckkissen gespannt werden, welche eine dorsal gerichtete Kraft auf die Handballen ausüben.

Die Erfindung hat sich zur Aufgabe gestellt, ein Behandlungsgerät zur Dehnung des Karpalkanals der menschlichen Hand anzugeben, welches eine verbesserte, zielgerichtetere Einleitung der zur Dehnung erforderlichen Kräfte ermöglicht.

Die Aufgabe wird gelöst durch die Merkmale des Anspruchs 1. Vorteilhafte Weiterbildungen sind den abhängigen Ansprüchen zu entnehmen.

Das erfindungsgemäße Behandlungsgerät umfasst zwei Adhäsivelemente in Form von Klebepads, welche geeignet sind, mit menschlicher Haut in Kontakt zu treten und ausgebildet sind, seitlich des Karpalkanals auf die Handballen einer zu behandelnden menschlichen Hand aufgeklebt zu werden. Weiter ist eine Dehnvorrichtung vorgesehen, welche ausgebildet ist, mit den Adhäsivelementen verbunden zu werden, um eine im Wesentlichen lateral gerichtete Dehnkraft zielgerichtet und direkt auf die Handballen seitlich des Karpalkanals zu übertragen. Zur Behandlung können die Adhäsivelemente beidseitig des Karpalkanals auf den daumenseitigen Handballen und den handkantenseitigen Handballen aufgeklebt werden. Die mittels der Dehnvorrichtung auf die Adhäsivelemente ausgeübte, lateral gerichtete Dehnkraft wird auf diese Weise zielgerichtet direkt -nurauf die Handballen seitlich des Karpalkanals übertragen.
Die Adhäsivelemente sind vorzugsweise als mindestens einseitig mit einer selbstklebenden Beschichtung versehene Träger ausgebildet. Wenn diese Träger auf den gegenüberliegenden Seiten mit einem reversiblen Schnellverschluss, vorzugsweise Klettverschluss, versehen sind, kann die Dehnvorrichtung auf besonders einfache Weise abnehmbar gestaltet werden.
Die Adhesivelemente können in Form von Klebepads ausgeführt werden, die in Größe und/oder Form an die Handballen einer zu behandelnden menschlichen Hand angepasst sind. Hierbei können auch unterschiedliche Größen von Klebepads für unterschiedliche Handgrößen vorgehalten werden, unter denen dann im konkreten Behandlungsfall eine Auswahl getroffen werden kann.
Bei einer ersten Ausführungsform weist die Dehnvorrichtung zwei Backen auf, welche gegeneinander aufgespannt werden können. Das Aufspannen kann beispielsweise mit einer Gewindespindel erfolgen, über welche sich die Backen gegeneinander abstützen. Somit kann auf einfache Weise die Dehnkraft den Bedürfnissen des Anwenders bzw. Patienten angepasst werden. Die Möglichkeit der individuellen Einstellung der Dehnkraft ist vorteilhaft, da das Schmerzempfinden der Anwender sehr unterschiedlich und zeitlich schwankend sein kann.

Durch den einfachen Aufbau kann die Dehnvorrichtung sehr klein und kompakt ausgeführt werden, so dass sie vom Benutzer einfach mitgeführt und wann immer sich die Gelegenheit bietet angewendet werden kann.

Oberhalb der Gewindespindel können die Backen über mindestens ein Gelenk miteinander verbunden sein. Dieses obere Gelenk erzeugt einen Gegendruck, der dafür sorgt, dass die Dehnkraft horizontal in der Ebene der Handfläche auf das zu dehnende Karpalband übertragen wird.

An ihrer Unterseite können die Backen jeweils eine Auflagefläche aufweisen, die einen reversiblen Schnellverschluss, vorzugsweise Klettverschluss, trägt. Die Dehnvorrichtung kann somit auf einfache Weise abnehmbar gestaltet werden.

Vorzugsweise ist die Kontur der Auflageflächen der beiden Backen individuell auf die Ballenform der zu behandelnden Hand angepasst. Dies ermöglicht eine weitere Verbesserung der Krafteinleitung. Hierzu kann beispielsweise ein Abdruck der zu behandelnden Hand genommen werden, nach dem die Auflageflächen individuell hergestellt werden können. Entsprechende anatomisch geformte Auflageflächen können natürlich auch für verschiedene typische Handformen und Handgrößen vorgefertigt sein, aus denen dann eine individuelle Auswahl erfolgen kann.

Bei einer alternativen Ausführungsform des Behandlungsgeräts weist die Dehnvorrichtung ein vorzugsweise elastisches Dehnband auf, welches derart ausgebildet ist, dass es über den Handrücken der zu behandelnden Hand gelegt werden kann um die Dehnkraft auf die Adhäsivelemente auszuüben. Mittels einer derartigen, als Bandage ausgeführten Dehnvorrichtung wird eine definierte Gegenkraft auf den Handrücken erzeugt, die über das Dehnband auf die Adhäsivelemente übertragen wird. Die mittels eines derartigen Dehnbandes übertragbare Kraft ist zwar geringer als im Falle der ersten Ausführungsform, kann dafür aber über eine längere Zeit erzielt werden. Eine derartige Bandage ist gut für einen Dauergebrauch geeignet, da sie den Anwender in seiner Bewegungsfähigkeit kaum einschränkt.

Um die Gegenkraft auf dem Handrücken zu erzeugen, kann in etwa mittig an dem Dehnband ein Polster angebracht sein. Das Polster kann insbesondere ein elastisches Druckpolster sein, beispielsweise ein Schaumkörper. Alternativ und/oder zusätzlich kann das Polster auch ein Luftkissen enthalten, welches mittels einer Handpumpe aufpumpbar ist.

Wenn an der Innenseite des Dehnbandes endseitig jeweils reversible Schnellverschlüsse, vorzugsweise Klettverschlüsse vorgesehen sind, so ist das Dehnband schnell abnehmbar. Außerdem kann über derartige Schnellverschlüsse auf einfache Weise die Dehnkraft angepasst werden. Darüber hinaus ist es auch möglich, mehrere unterschiedliche Dehnbänder mit unterschiedlichen elastischen Eigenschaften vorzusehen, aus denen zur Anpassung der individuell geeigneten Dehnkraft das geeignete ausgewählt werden kann.

Eine derartige Dehnungsbandage kann sehr kostengünstig in hohen Stückzahlen und in unterschiedlichen Größen hergestellt werden, so dass ein Aufwand für individuelle Anpassung entfällt. Ein derartiges Behandlungsgerät bietet sich damit ideal zur Eigenbehandlung durch betroffene Patienten an.

Weitere Vorteile und Eigenschaften der vorliegenden Erfindung werden im Folgenden anhand der Figuren und anhand von Ausführungsbeispielen erläutert. Dabei zeigt:
- Figur 1: eine menschliche Hand mit erfindungsgemäß angebrachten Adhäsivelementen,
- Figur 2a: eine Draufsicht auf eine Dehnvorrichtung in einem ersten Ausführungsbeispiel,
- Figur 2b: einen Längsschnitt durch die Dehnvorrichtung des ersten Ausführungsbeispiels, und
- Figur 3: einen Längsschnitt durch eine als Bandage ausgeführte Dehnvorrichtung in einem zweiten Ausführungsbeispiel.

Die erfindungsgemäße Lösung beruht auf der Dehnung des Karpalbandes durch Übertragung einer lateral gerichteten Dehnkraft auf die Handballen der zu behandelnden Hand. Als Ziel wird hier eine Dehnung um mehrere Millimeter angestrebt, welche im Ergebnis vergleichbar mit der bei einem chirurgischen Eingriff erreichten Erweiterung von etwa 3 bis 4 mm ist. Dabei soll die Dehnkraft möglichst horizontal in der Ebene der Handfläche auf das Karpalband übertragen werden. Dies wird dadurch erreicht, dass Adhäsivelemente vorgesehen sind, welche in der Art eines Pflasters auf die Haut der Handballen aufgeklebt werden können.

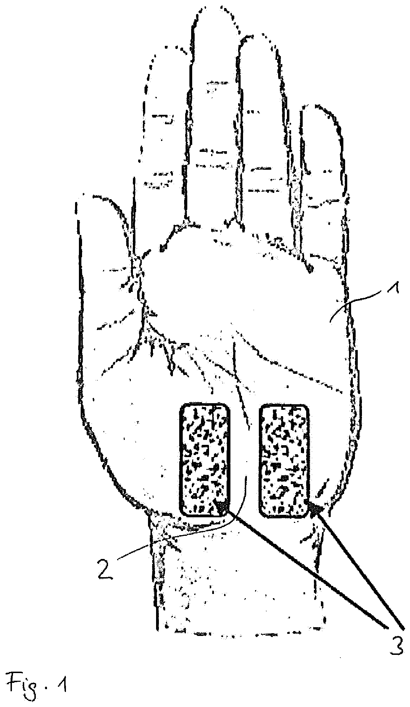

In Figur 1 ist die Handinnenseite einer Hand 1 gezeigt, bei der seitlich des Verlaufs des Karpalkanals 2 zwei Adhäsivelemente 3 auf die Handballen aufgeklebt sind. Bei den Adhäsivelementen 3 handelt es sich um Klebepads, welche auf der Seite, die mit der Haut in Kontakt steht, mit einer hautverträglichen, selbstklebenden Beschichtung versehen sind. Auf der Oberseite der Klebepads 3 befindet sich der mit Schlaufen versehene Teil eines Klettverschlusses. Die Klebepads werden auf die Haut aufgeklebt und ermöglichen eine zuverlässige und zielgerichtete Übertragung einer Dehnkraft von einer entsprechenden Dehnvorrichtung.

Die Klebepads sind als Einmalartikel ausgelegt, können jedoch bis zu mehrere Tage auf der Hand verbleiben und ermöglichen so eine Vielzahl von Behandlungen. Sind die Klebepads verschmutzt oder lösen sich ab, so werden sie durch neue ersetzt.

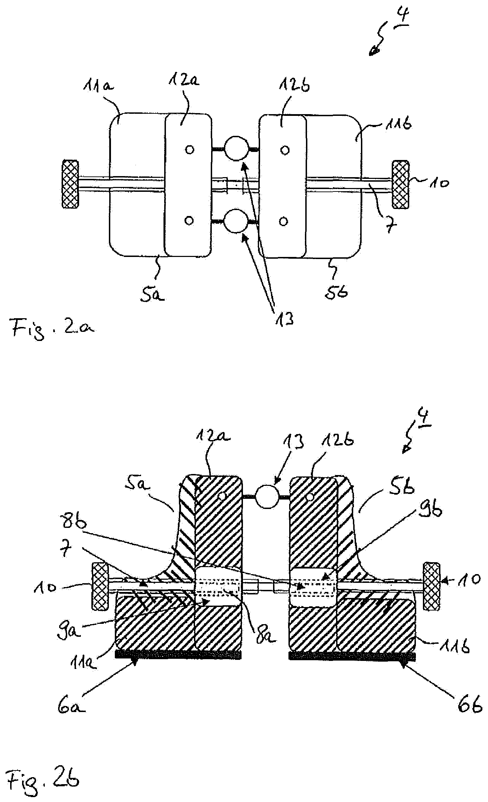

In den Figuren 2a und 2b ist eine Dehnvorrichtung dargestellt, mittels der eine Dehnkraft auf die Klebepads übertragen werden kann. Die Dehnvorrichtung 4 besitzt zwei seitliche Backen 5a, 5b, auf deren Unterseite im Bereich einer handseitigen Anlagefläche die Hakenseite eines Klettverschlusses 6a, 6b angebracht ist. Mittels einer Gewindespindel 7 können die Backen 5a, 5b in der Art eines Schraubstocks auseinanderbewegt werden. Die Gewindespindel 7 ist im Ausführungsbeispiel als durchgehende Gewindestange ausgeführt, wobei diese auf der linken Seite mit einem Linksgewinde 8a und auf der rechten Seite mit einem Rechtsgewinde 8b versehen ist. Entsprechend weist der linke Backen 5a eine Gewindehülse 9a mit Linksgewinde und der rechte Backen 5b eine Gewindehülse 9b mit Rechtsgewinde auf. An beiden Seiten der Gewindespindel 7 befindet sich ein Rändelschraubenkopf 10, so dass eine beidseitige Betätigung möglich ist.

Im unteren Bereich verbreitern sich die beiden Backen 5a, 5b zu einem Sockel 11a, 11b der die handseitige Anlagefläche bildet. Im oberen Bereich erstrecken sich die beiden Backen 5a, 5b in Form von Schenkeln 12a, 12b im Wesentlichen senkrecht zu den handseitigen Anlageflächen 6a, 6b. An ihrem oberen Ende sind die Schenkel 12a, 12b über zwei Gelenke 13 miteinander verbunden. Die Gelenke 13 dienen zur Kompensation des Gegendrucks und Bewirken so eine im Wesentlichen laterale Kraftübertragung auf die Klebepads 3.

Der Mechanismus zum Aufspreizen der Backen 5a, 5b, lässt sich in vielfältiger Weise abwandeln. Beispielsweise kann die Gewindespindel 7 in der Mitte geteilt und drehbar verbunden werden, so dass die Gewinde auf beiden Seiten als Rechtsgewinde ausgeführt werden können. Ebenso wäre es möglich, eine Gewindespindel durch nur einen der beiden Backen 5a, 5b zu führen, welche sich am anderen Backen abstützt und dort drehbar gelagert ist. Auch eine Vielzahl anderer Spreizmechanismen, wie beispielsweise ein Scherenmechanismus sind an dieser Stelle möglich. Genauso können die Backen auch durch ein zwischen den Backen angeordnetes Luftkissen, welches über eine Handpumpe aufgepumpt werden kann, auseinandergespreizt werden. Um einen besseren Halt der Dehnvorrichtung 4 auf den Klebepads zu gewährleisten kann an den Backen 5a, 5b zusätzlich ein Halteband angebracht sein, welches über den Handrücken gelegt werden kann.

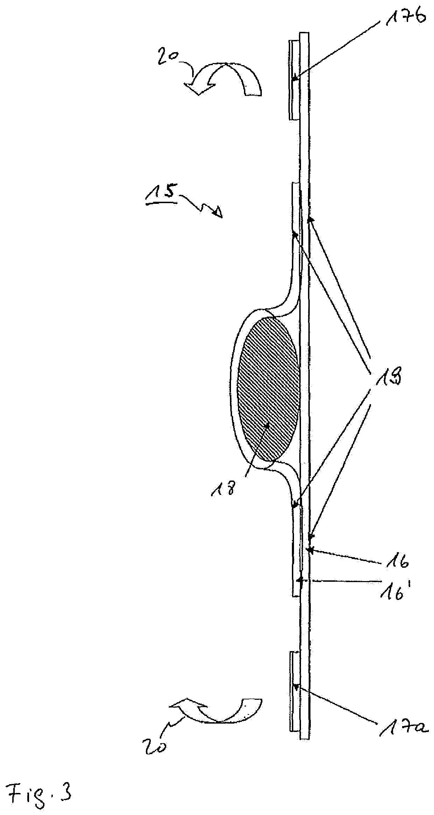

Ein zweites Ausführungsbeispiel für eine Dehnvorrichtung ist in Figur 3 dargestellt. Die Dehnvorrichtung 15 ist hier in der Art einer Bandage ausgeführt, welche ein Dehnband 16 aus elastischem Textilgewebe umfasst. Auf der Innenseite des Dehnbandes 16 ist an beiden Enden wieder die Hakenseite eines Klettverschlusses aufgenäht. Etwa in der Mitte des Dehnbandes 16 befindet sich ebenfalls auf der Innenseite ein Polster 18, welches im Ausführungsbeispiel als elastischer Schaumkörper ausgeführt ist. Das Polster 18 ist dabei über eine weitere Lage elastischen Textilgewebes 16' auf der Innenseite des Dehnbandes 16 aufgenäht. Der vernähte Bereich ist dabei schematisch mit dem Bezugszeichen 19 bezeichnet.

Die Bandage 15 wird in Richtung der angedeuteten Pfeile 20 um die Hand gelegt, so dass das Polster 18 auf dem Handrücken zu liegen kommt. Die Klettverschlüsse 17a, 17b werden dann an den auf der Handinnenseite aufgeklebten Klebepads 3 befestigt. Die Klettverschlüsse ermöglichen dabei auf einfache Weise die Spannung des Dehnbandes und damit die Dehnkraft zu regulieren. Das Polster 18 auf dem Handrücken sorgt durch seine elastische Eigenschaft für ausreichend Gegenzug auf dem Handrücken. Das Polster 18 kann zusätzlich oder ersatzweise ein Luftpolster enthalten, welches durch Aufpumpen mittels einer Handpumpe eine Regulierung der Dehnkraft, welche auf die Klebepads übertragen wird, ermöglicht. Das Polster 18 unterstützt somit die Dehnung des Karpalkanals.

Durch die Klettklebepads kann die elastische Bandage 15 bei Bedarf abgenommen und wieder befestigt werden. Das Material der Bandage ist ähnlich einer elastischen Binde und kann in verschiedenen Elastizitäten gefertigt werden. Eine Steigerung der Behandlung ist dadurch entsprechend dem Dehnungsverlauf möglich. Durch unterschiedliche Längen der Bandage kann die Dehnvorrichtung dem individuellen Umfang des Handgelenks angepasst werden. Die Feinjustierung wird über den Klettverschluss vorgenommen.

## Patentansprüche

1. Behandlungsgerät zur Dehnung des Karpalkanals (2) der menschlichen Hand (1), **gekennzeichnet durch** zwei Adhäsivelemente (3) in Form von Klebepads, welche geeignet sind, mit menschlicher Haut in Kontakt zu treten und ausgebildet sind, seitlich des Karpalkanals (2) auf die Handballen einer zu behandelnden menschlichen Hand (1) aufgeklebt zu werden, und durch eine Dehnvorrichtung (4), welche ausgebildet ist, mit den Adhäsivelementen (3) verbunden zu werden, um eine im Wesentlichen lateral gerichtete Dehnkraft zielgerichtet und direkt auf die Handballen seitlich des Karpalkanals (2) zu übertragen.

2. Behandlungsgerät nach Anspruch 1,
bei dem die Adhäsivelemente (3) als mindestens einseitig mit einer selbstklebenden Beschichtung versehene Träger ausgebildet sind.

3. Behandlungsgerät nach Anspruch 2,
bei dem die Träger auf der der selbstklebenden Beschichtung gegenüberliegenden Seite mit einem reversiblen Schnellverschluss, vorzugsweise Klettverschluss, versehen sind.

4. Behandlungsgerät nach einem der vorangehenden Ansprüche,
bei dem die Dehnvorrichtung (4) zwei Backen (5a, 5b) umfasst, welche gegeneinander aufgespannt werden können.

5. Behandlungsgerät nach Anspruch 4,
bei dem sich die Backen (5a, 5b) über mindestens eine Gewindespindel (7) gegeneinander abstützen.

6. Behandlungsgerät nach Anspruch 5,
bei dem die Backen (5a, 5b) oberhalb der Gewindespindel (7) über mindestens ein Gelenk (13) miteinander verbunden sind.

7. Behandlungsgerät nach einem der Ansprüche 4 bis 6,
bei dem die Backen (5a, 5b) an ihrer Unterseite jeweils eine Auflagefläche (6a, 6b) aufweisen, die einen reversiblen Schnellverschluss, vorzugsweise Klettverschluss, trägt.

8. Behandlungsgerät nach Anspruch 5,
bei dem die Kontur der Auflagefläche (6a, 6b) individuell auf die Ballenform der zu behandelnden Hand (1) angepasst ist.

9. Behandlungsgerät nach einem der Ansprüche 1 bis 3,
bei dem die Dehnvorrichtung als Bandage ausgebildet ist und ein vorzugsweise elastisches Dehnband (16) umfasst, welches derart ausgebildet ist, dass es über den Handrücken der zu behandelnden Hand (1) gelegt werden kann um die Dehnkraft auf die Adhäsivelemente (3) zu übertragen.

10. Behandlungsgerät nach Anspruch 8,
bei dem das Dehnband (16) eine Innenseite und eine Außenseite aufweist und an der Innenseite endseitig jeweils einen reversiblen Schnellverschluss (17a, 17b), vorzugsweise Klettverschluss vorgesehen ist.

11. Behandlungsgerät nach Anspruch 8 oder 9,
bei dem das Dehnband (16) in etwa mittig ein Polster (18) trägt.

12. Behandlungsgerät nach Anspruch 10,
bei dem das Polster (18) ein elastisches Druckpolster ist.

13. Behandlungsgerät nach Anspruch 10 oder 11,
bei dem das Polster (18) als elastischer Schaumkörper ausgebildet ist.

14. Behandlungsgerät nach Anspruch 10 oder 11,
bei dem das Polster (18) ein Luftkissen enthält, welches mittels einer Handpumpe aufpumpbar ist.

## Claims

1. Treatment device for stretching the carpal tunnel (2) of the human hand (1), **characterised by** two adhesive elements (3) in the form of adhesive pads which are suitable for entering into contact with human skin and are arranged to be adhesively bonded, laterally of the carpal tunnel (2), to the thenar and hypothenar eminences of a human hand (1) to be treated, and by a stretching device (4) which is arranged to be joined to the adhesive elements (3) in order to transmit a substantially laterally directed stretching force specifically and directly to the thenar and hypothenar eminences laterally of the carpal tunnel (2).

2. Treatment device according to claim 1,
wherein the adhesive elements (3) are in the form of straps which are provided with a self-adhesive coating on at least one side.

3. Treatment device according to claim 2,
wherein the straps are provided with a reversible quick-release fastener, preferably a hook-and-loop fastener, on the side opposite the self-adhesive coating.

4. Treatment device according to either one of the preceding claims,
wherein the stretching device (4) comprises two jaws (5a, 5b) which can be clamped relative to one another.

5. Treatment device according to claim 4,
wherein the jaws (5a, 5b) are supported against one another by means of at least one threaded spindle (7).

6. Treatment device according to claim 5,
wherein the jaws (5a, 5b) are connected to one another above the threaded spindle (7) by way of at least one joint (13).

7. Treatment device according to any one of claims 4 to 6,
wherein the jaws (5a, 5b) each have, on their underside, a contact surface (6a, 6b) which carries a reversible quick-release fastener, preferably a hook-and-loop fastener.

8. Treatment device according to claim 5,
wherein the contour of the contact surface (6a, 6b) is matched individually to the shape of the eminences of the hand (1) being treated.

9. Treatment device according to any one of claims 1 to 3,
wherein the stretching device is in the form of a bandage and comprises a stretch tape (16), preferably an elastic stretch tape, which is constructed in such a way that it can be laid over the back of the hand (1) being treated in order to transmit the stretching force to the adhesive elements (3).

10. Treatment device according to claim 8,
wherein the stretch tape (16) has an inner side and an outer side, and on the inner side a reversible quick-release fastener (17a, 17b), preferably a hook-and-loop fastener, is provided at each end.

11. Treatment device according to claim 8 or 9,
wherein the stretch tape (16) has a pad (18) approximately in the centre.

12. Treatment device according to claim 10,
wherein the pad (18) is a resilient pressure pad.

13. Treatment device according to claim 10 or 11,
wherein the pad (18) is formed as a resilient foam body.

14. Treatment device according to claim 10 or 11,
wherein the pad (18) contains an air cushion which is inflatable by means of a hand pump.

## Revendications

1. Appareil de traitement dévolu à l'étirement du canal carpien (2) de la main humaine (1), **caractérisé par** deux éléments adhérents (3) revêtant la forme de patins d'adhésif aptes à entrer en contact avec une peau humaine et réalisés pour être collés sur les régions palmaires d'une main humaine (1) à traiter, sur les côtés du canal carpien (2) ; et par un dispositif d'étirement (4) réalisé pour être relié auxdits éléments adhérents (3) en vue de transmettre auxdites régions palmaires, de manière ciblée et directe, sur les côtés dudit canal carpien (2), une force d'étirement dirigée pour l'essentiel dans le sens latéral.

2. Appareil de traitement selon la revendication 1,
dans lequel les éléments adhérents (3) sont réalisés sous la forme de substrats munis, au moins sur une face, d'un revêtement auto-adhésif.

3. Appareil de traitement selon la revendication 2,
dans lequel les substrats sont pourvus d'une fermeture rapide libérable, de préférence d'une fermeture à auto-agrippage, sur la face pointant à l'opposé du revêtement auto-adhésif.

4. Appareil de traitement selon l'une des revendications précédentes,
dans lequel le dispositif d'étirement (4) inclut deux mâchoires (5a, 5b) pouvant être serrées l'une contre l'autre.

5. Appareil de traitement selon la revendication 4,
dans lequel les mâchoires (5a, 5b) prennent appui l'une contre l'autre par l'intermédiaire d'au moins une broche filetée (7).

6. Appareil de traitement selon la revendication 5,
dans lequel les mâchoires (5a, 5b) sont reliées l'une à l'autre, au-dessus de la broche filetée (7), par l'intermédiaire d'au moins une articulation (13).

7. Appareil de traitement selon l'une des revendications 4 à 6,
dans lequel les mâchoires (5a, 5b) sont respectivement dotées, à leur face inférieure, d'une surface de contact (6a, 6b) qui porte une fermeture rapide libérable, de préférence une fermeture à auto-agrippage.

8. Appareil de traitement selon la revendication 5,
dans lequel le profil de la surface de contact (6a, 6b) est adapté individuellement à la forme de la paume de la main (1) à traiter.

9. Appareil de traitement selon l'une des revendications 1 à 3,
dans lequel le dispositif d'étirement est réalisé sous la forme d'un pansement et inclut une sangle étireuse (16), de préférence élastique, réalisée de façon telle qu'elle puisse être mise en place au-dessus du dos de la main (1) à traiter, afin de transmettre la force d'étirement aux éléments adhérents (3).

10. Appareil de traitement selon la revendication 8,
dans lequel la sangle étireuse (16) comprend une face intérieure et une face extérieure, une fermeture rapide (17a, 17b) libérable, de préférence une fermeture à auto-agrippage, étant respectivement prévue aux extrémités de ladite face intérieure.

11. Appareil de traitement selon la revendication 8 ou 9,
dans lequel la sangle étireuse (16) porte un rembourrage (18) à emplacement sensiblement central.

12. Appareil de traitement selon la revendication 10,
dans lequel le rembourrage (18) est un rembourrage presseur élastique.

13. Appareil de traitement selon la revendication 10 ou 11,
dans lequel le rembourrage (18) est réalisé sous la forme d'un corps élastique en mousse.

14. Appareil de traitement selon la revendication 10 ou 11,
dans lequel le rembourrage (18) renferme un coussin d'air pouvant être gonflé au moyen d'une pompe manuelle.
